# NEW EUROPEAN PATENT SPECIFICATION

(11) **EP 0 702 529 B2**
(45) Date of publication and mention of the opposition decision: **29.10.2008**
(45) Mention of the grant of the patent: 23.10.2002
(21) Application number: 94918102.8
(22) Date of filing: 23.05.1994
(51) Int. Cl.: A61B 17/12, A61F 5/00

(54) **UNIVERSAL GASTRIC BAND**
UNIVERSELLES MAGENBAND
CEINTURE GASTRIQUE UNIVERSELLE

(30) Priority: 27.05.1993 US 68411
(43) Date of publication of application: 27.03.1996
(60) Divisional application: 01126574.1
(73) Proprietor: ALLERGAN, INC., Irvine CA 92612 (US)
(72) Inventor: VINCENT, Vernon, L., Santa Barbara, CA 93105 (US)
(74) Representative: Becher, Claus Thomas
(86) International application number: PCT/US1994/005814
(87) International publication number: WO 1994/027504

(56) References cited:
- EP-A- 0 611 561
- WO-A-86/04498
- US-A- 4 592 339
- US-A- 4 813 416
- US-A- 5 074 868
- US-A- 5 152 770
- US-A- 5 207 694
- US-A- 5 226 429

## Description

This invention relates to devices for the control of obesity, and, more particularly, to a surgically implantable adjustable band for encircling the stomach.

Several years ago, Kuzmak, et al. in US-A-4 592 339 described a belt-like band for encircling the stomach to control morbid obesity. The band comprised substantially a belt which could be passed around the stomach in a circling position and then cinched tight in order to adjust the stoma opening within the stomach. Further improvements in the gastric band have included having an adjustable portion of the band comprising an inflatable member which permits the fine adjustment of the stoma opening after the size of the stoma is initially set by the band tightening procedure. The band tightening procedure normally involves the placement of a calibrating apparatus in the stomach to detect the stoma size. Once the apparatus is placed within the stomach the gastric band is cinched down tight until the stoma opening approximates the desired size. The band is fastened in position, usually by sutures, and the stoma opening finally adjusted by injecting a fluid into an inflatable member which is coextensive with a portion of the inner stomach-contacting surface of the band. The means for injecting the fluid into the inflatable member usually comprises a fill port located beneath the skin which can be accessed extracorporeally by transdermal injection. Thus, following implantation, the gastric band can be adjusted, within a narrow range, to enlarge or reduce the stoma as required.

Furthermore, US-A-5 074 868 discloses a gastric banding device for creating a stoma opening in the stomach for restricting food intake into the lower digestive portion of the stomach. The band is operatively placed to encircle the stomach. Once in position, it is held securely with sutures on the outside of the stomach thereby prohibiting the encircled stoma opening from expanding. The suturing step is facilitated by a suture hole located on the tail end of the device. The head end of the device comprises a slot through which the tail end is to be inserted. The slot on the head end is no longer needed when the band is secured in a circle by the application of sutures or by a cotter-type of pin and is cut away. This known gastric band is said to be removable without the need for major surgery. This document is silent about the band being adapted for laparoscopic placement.

EP 0 611 561 A1 - which is a document to be considered only within the meaning of Article 54(3) EPC - discloses a gastric banding device for regulating the size of a stoma opening in the stomach of a patient. The device includes an elongate band having a portion that encircles the stomach so as to limit the food intake by the digestive portion of the stomach. Furthermore, this device is said to be implanted and removed using laparoscopic procedures. A locking member protruding from a location near the distal end of the encircling band portion is inserted into a recess formed in a receiving segment at an intermediate location along the encircling band portion so as to form a circle or loop of fixed diameter. A movable bolt secures the locking member in the recess. The bolt is said to be displaced remotely, thus releasing the locking member and allegedly allowing laparoscopic removal of the banding device. The circumference of the stomach encircling band portion is covered by an expandable section that is intended to be in contact with the surface of the stomach. The size of the stoma opening can be adjusted by injecting into or removing fluid from the expandable section.

WO 86/04498 relates to a surgical device for banding of the stomach of a patient, such as a patient suffering from obesity, which device comprises a band arranged to be joined into a ring about the stomach and provided with a lumen filled with a fluid, in which lumen the amount of fluid can be changed via an injection port placed under the skin of the patient, thus that the cross sectional area of the ring can be varied. The joining is done by overlapping the free band ends and suturing them together.

US-A-5 207 694 relates to a method, a kit and an applicator for performing a surgical occlusion and is used instead of conventional metal clamps. A tubular applicator open at both ends is introduced with the first end adjacent the free-prepared tissue. At least one bundle-strap type tie is introduced into the applicator. A tie portion protruding from the first end of the applicator is caught in order to position the tie in its proper place around the tissue. Thereafter, the tie is formed into a locked occlusion loop around the tissue. The occlusion technique is said to be applicable to both laparotomy and laparoscopy.

One of the disadvantages of the prior art gastric bands is the difficulty in tensioning the band around the stomach to approximate the desired stoma. This is particularly difficult when the band is to be placed laparoscopically. When the band is placed laparoscopically, special instruments are required to tension the band prior to the fine adjustment of the stoma. Such instruments as are required to grasp the band and pull it into an encircling position around the stomach then cinch it tight, are difficult to manipulate through a laparoscopic canula. It would therefore be desirable to provide a band having an inflatable member thereon which can be easily fastened into an encircling position around the stomach and in which the adjustment of the stoma opening can be regulated solely by the injection of fluid into the inflatable member.

An object of this invention is to provide a gastric band which can be laparoscopically placed and non-adjustably fastened into an encircling position around the stomach to form a ring having an inner diameter.

It is yet another object of this invention to provide a gastric band wherein the band can be easily affixed in an encircling position around the stomach by conventional laparoscopic instruments.

It is yet another object of this invention to provide a gastric band adapted for laparoscopic placement around the stomach and thereafter to be non-adjustably locked into a circle around the stomach to compress the encircled portion of the stomach to constrict the stoma thereof which constriction may be further adjusted solely by means of an inflatable member.

It is yet a further object of the invention to provide a gastric band having an inflatable member deployed on an inner surface wherein said inner surface presents a smooths continuous surface when the band is fastened in an encircling position and the inflatable member is inflated.

These objects are achieved with the features of the claims and will soon become apparent as we turn now to the preferred embodiments which are illustrated in the following drawing, in which:
Figure 1 is a perspective view of the gastric band according to the present invention.
Figure 2 is the gastric band according to the present invention fastened in an encircling position and partially inflated.

As mentioned earlier, prior art gastric bands have two steps or stages of adjustment: the first step roughly affixes the gastric band in a position to encircle the stomach with the stoma opening approximating the desired size. This first adjustment is analogous to tightening a belt around the waist until the buckle tongue passes through one of a plurality of holes rendering comfortable tension. The next step in the adjustment requires injection of a fluid into the inflatable member to bring the stoma opening to the desired size.

Prior art gastric bands, lacking a large range of inflatable adjustment, require an initial "rough" adjustment. The "rough" adjustment comprising the first step in the gastric stoma-constricting process requires tensioning the prior art band, and holding the tension on the band, while sutures are placed to lock the band in the encircling position. This is a cumbersome procedure to perform laparoscopically and the design of the present invention overcomes some of the difficulties with tensioning the band.

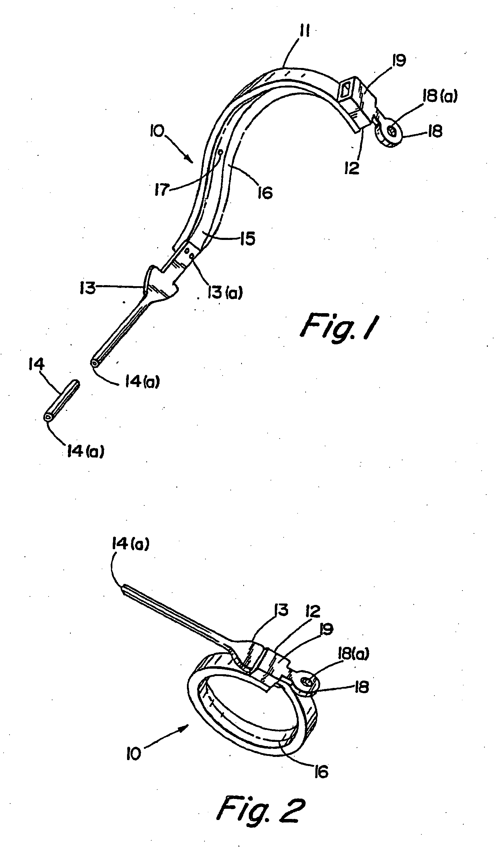

Turning now to Figure 1, we see a gastric band, generally indicated at the numeral 10, which has a body portion 11 with an inner stomach-facing surface 15. The body portion 11 has a head end 12 and a tail end 13 with one or more suture holes 13(a) therein. A fill tube 14, which is generally a tube having a single lumen 14(a) coextensive therewith, is in fluid communication with an inflatable member 16 on the inner surface 15 of the band body 11. It is an important feature of this invention that the inflatable portion 16 is substantially co-extensive with the inner surface 15 of the body portion 11 so as to present a substantially continuous surface free of abrupt contours when the band encircles the stomach and the inflatable portion 16 is inflated. The central lumen 14(a) of the fill tube 14 enters into the inflatable member 16 at lumen opening 17. The head 12 of the body portion 11 has a buckle 19 with a pull tab 18 having a suture hole 18(a) integral therewith. Both portions which receive a suture, that is the pull tab 18 and the tail 13, are preferably medical grade silicone reinforced with dacron.

In practice, the gastric band is placed in the circling position around the stomach as shown in Figure 2. (In Figure 2 the stomach is omitted for clarity.) This is accomplished by pushing the fill tube 14 through a laparoscopic canula (not shown) in the patient's abdomen. Laparoscopic placement consists of blunt dissection around the greater curvature of the stomach. The end of the fill tube 14 is passed around the stomach, and the tail 13 is attached to the buckle 19, so that the buckle and the tail are non-adjustably and irreversibly affixed to one another. In this sense, the band is a "one-size-fits-all" device. That is, for a particular band there is only one single position in which the tail and buckle can be attached to one another. Further adjustment of the stoma; the narrow opening in the stomach created by the band, is performed solely by inflation or deflation of the inflatable member 16 after the band is secured in this single position.

Prior art gastric bands employ an adjustable balloon portion used for small post-operative adjustment of the stoma if necessary. The laparoscopic hydraulic gastric band outlined herein has a larger balloon covering a greater area of the inner surface of the gastric band than taught in the prior art. The inflatable member or balloon 16 preferably is coextensive with the inner stomach-facing surface 15 of the band between the head end 12 and the tail end 13. This larger balloon 16 is utilized to adjust the stoma at the time of surgery. The interior of the adjustable balloon 16 is in fluid communication with an injection reservoir (not shown) by means of the central lumen 14(a) of the fill tube 14, as with prior art adjustable gastric bands. The inflatable member 16 is gradually inflated with saline via the injection reservoir (not shown) such that the inflatable member 16, which, as stated above, is coextensive with the inner surface 15 of the band 11 between the head end 12 and the tail end 13, presses on and constricts the stomach wall underlying the band. This results in the decrease of the opening (stoma) inside the stomach directly under the encircling band. The continuous surface presented by the enlarged inflatable member to the underlying tissue inhibits squeezing of the tissue into wrinkles, dimples or similar discontinuities such as are present on the inner surface of prior art bands and thereby prevents necrosis.

An electronic pressure transducer known as a gastrostenometer electronic sensor, coupled to a calibration probe known generically as a "calibration tube" is used to measure the size of the stoma. The calibration tube is introduced through the patient's mouth and esophagus into the stomach. The band is inflated, tightening it around the tip of the calibration tube. The gastrostenometer electronic sensor indicates the stoma diameter created as the band is tightened.

The present invention allows for the laparoscopic implantation of a therapeutic surgical device without the risks associated with major abdominal incisions in the obese patient. Faster healing, reduced complications, reduced hospital stay and drastically reduced pain are among the advantages of laparoscopic placement. The gastric band according to the present invention facilitates laparoscopic placement and will also allow wider use of the gastric band system to create a gastric restricter procedure for the treatment of morbid obesity. This band enables more surgeons, skilled in the art of laparoscopic surgery to undertake this operation without the need for highly specialized, difficult to use tensioning instruments. Since there is only one single, non-adjustable locking position for the band around the stomach, the procedure itself should lend itself to more rigorous standardization. Also, more patients will consider having this procedure performed because of the advantages of laparoscopic surgery over traditional abdominal laparotomies. These advantages, coupled with the advantages of the prior art gastric bands, which enable post operative fine tuning of the stoma, enable a more facile procedure to be performed.

In general, the smoother or more continuous the inner surface 15 of the band 10, the less likely the chance of necrosis. The single locking or fastening position of the present gastric band ensures that the inflatable member 16 will be substantially coextensive with the inner surface 15 of the band 10 and continuous with the buckle 19. This fixed-diameter band enables the inner surface 15 to present as a continuous surface to the encircled tissue as the band is inflated, thereby preventing necrosis. Prior art gastric bands lack a provision for assuring that the inflatable member is adjacent to and continuous with the buckle portion when the band is locked into an encircling position. This permits a gap or space between the inflatable member and the buckle along the inner surface when the inflatable member is inflated. Tissue can squeeze into such a gap, causing necrosis. The reduction and/or elimination of such discontinuities on the inner surface of the (inflated) band as taught hereinabove reduces the possibility of necrosis.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A gastric band (10) for treatment of morbid obesity, comprising a body portion (11) having a head end (12) and a tail end (13) and an inner stomach-facing surface (15) therebetween,
said tail end (13) comprising an elongate tubular member (14) capable of fluid tight connection to an injection reservoir,
said head end (12) comprising thereon a buckle (19) for receiving said tail end (13) and for locking said gastric band (10) non-adjustably into a circle having an inner circumference,
an inflatable member (16) on the inner surface (15) being in fluid communication with said tubular member (14), the inflatable member (16) being coextensive with said inner surface (15) of said body portion (11) when said gastric band (10) is non-adjustably locked into said circle,
said gastric band (10) being adapted for laparoscopic placement around the stomach of a patient.

2. The gastric band (10) of claim 1, wherein said inflatable member (16) does not wrinkle or fold, thereby presenting a substantially smooth contour along said inner circumference.

## Patentansprüche

1. Magenband (10) zur Behandlung von pathologischer Fettleibigkeit, umfassend einen Körperabschnitt (11) mit einem Kopfende (12) und einem Schwanzende (13) und dazwischen einer dem Magen zugewandten Innenfläche (15), wobei
das Schwanzende (13) ein längliches, röhrenförmiges Element (14) mit der Fähigkeit zu einer fluiddichten Verbindung mit einem Injektionsreservoir aufweist,
das Kopfende (12) auf ihm eine Schnalle (19) zum Aufnehmen des Schwanzendes (13) und zum nichtverstellbaren Schließen des Magenbandes (10) zu einem Kreis mit einem Innenumfang aufweist,
ein aufblasbares Element (16) auf der Innenfläche (15) mit dem röhrenförmigen Element (14) in Fluidkommunikation ist, das aufblasbare Element (16) mit der Innenfläche (15) des Körperabschnitts (11) koextensiv ist, wenn das Magenband (10) nichtverstellbar zu dem Kreis geschlossen ist,
das Magenband (10) zur laparoskopischen Anordnung um den Magen eines Patienten geeignet ist.

2. Magenband (10) nach Anspruch 1, wobei das aufblasbare Element (16) sich nicht knittert oder faltet, wodurch eine im Wesentlichen glatte Kontur entlang des Innenumfangs bereitgestellt wird.

## Revendications

1. Ceinture gastrique (10) pour le traitement de l'obésité pathologique, comprenant une portion de corps (11) avec une extrémité de tête (12) et une extrémité de queue (13) et entre celles-ci une surface intérieure (15) en regard de l'estomac,
ladite extrémité de queue (13) comprenant un élément tubulaire allongé (14) apte à une communication étanche au fluide avec un réservoir d'injection,
ladite extrémité de tête (12) comprenant sur celle-ci une boucle (19) pour recevoir ladite extrémité de queue (13) et pour verrouiller ladite ceinture gastrique (10) de façon non ajustable dans un cercle ayant une circonférence intérieure,
un élément gonflable (16) sur la surface intérieure (15) étant en communication de fluide avec ledit élément tubulaire (14), l'élément gonflable (16) étant de même étendue que ladite surface intérieure (15) de ladite portion de corps (11) lorsque ladite ceinture gastrique (10) est verrouillée de façon non ajustable dans ledit cercle,
ladite ceinture gastrique (10) étant apte à la mise en place laparoscopique autour de l'estomac d'un patient.

2. Ceinture gastrique (10) selon la revendication 1, dans laquelle ledit élément gonflable (16) ne se fronce ni ne fait des plis, présentant ainsi un contour sensiblement lisse le long de ladite circonférence intérieure.
